# FASCICULE DE BREVET EUROPEEN

(11) **EP 0 971 756 B1**
(45) Date de publication et mention de la délivrance du brevet: **17.12.2003**
(21) Numéro de dépôt: 98914943.0
(22) Date de dépôt: 18.03.1998
(51) Int. Cl.: A61M 1/10

(54) **COEUR ARTIFICIEL TOTALEMENT IMPLANTABLE**
KÜNSTLICHES HERZ FÜR DIE TOTALIMPLANTATION
ARTIFICIAL HEART CAPABLE OF BEING WHOLLY IMPLANTED

(30) Priorité: 19.03.1997 FR 9703349
(43) Date de publication de la demande: 19.01.2000
(73) Titulaire: CENTRE TECHNIQUE DES INDUSTRIES MECANIQUES, F-60300 Senlis (FR)
(72) Inventeur: WARTELLE, Claude, André, F-60270 Gouvieux (FR); DENAMUR, Alain, Marcel, Les Tilleuls, 60304 Senlis (FR)
(74) Mandataire: Bertrand, Didier
(86) Numéro de dépôt international: FR9800544
(87) Numéro de publication internationale: WO98041247

(56) Documents cités:
- FR-A- 2 625 903
- US-A- 4 058 857
- US-A- 4 750 903

## Description

L'invention concerne un coeur artificiel totalement implantable.

Le document EP-A-0 079 373 fait connaître une prothèse cardiaque totale dont les chambres ventriculaires sont actionnées par des pompes séparées du coeur lui-même. Il en résulte des pertes de charge importantes, un grand volume d'huile de pompage et un certain encombrement du système.

Une amélioration significative a été obtenue grâce à l'architecture décrite dans le document FR-A-2 625 903. Cette architecture, qui est à la base de la présente invention, se caractérise par un module monocoque abritant deux chambres ventriculaires indépendantes pourvues chacune de deux orifices munis de valve respectivement pour l'éjection et l'admission du sang, chaque chambre ayant son volume variable grâce à une membrane mobile constituant une paroi de la chambre et actionnée hydrauliquement par un fluide entraîné par un actionneur comprenant un moteur et une pompe volumétrique et intégré dans la coque.

Dans le coeur artificiel décrit dans le document, comme du reste dans celui décrit par EP-A- 0 079 373, les chambres ventriculaires se font face en formant un V dont la pointe est vers le bas, et entre les branches duquel sont disposés les orifices d'éjection et d'admission du sang, destinés à être raccordés aux organes naturels. L'extérieur du V est réservé aux chambres hydrauliques d'actionnement de la membrane, et aux groupes motopompes, en position appendiculaire.

US-A-4 750 903 énonce qu'il n'est pas possible de miniaturiser suffisamment un coeur à membrane pour le rendre vraiment implantable et propose un coeur d'un type différent, à poche.

Malgré tout son intérêt, le coeur artificiel à membrane peut encore être amélioré, sur le plan de la compacité et de la réduction des pertes de charge hydrauliques du côté de l'actionnement. Tel est l'objet de la présente invention.

La présente invention se distingue par une architecture qui prend le contre-pied des dispositions communément admises. Elle se caractérise essentiellement en ce que les chambres ventriculaires sont agencées de manière à former un V renversé, c'est-à-dire pointe vers le haut, entre les branches duquel sont disposés lesdits actionneurs et l'espace destiné au fluide d'actionnement.

Grâce à cette architecture révolutionnaire, on peut réduire l'encombrement total du coeur en supprimant la nécessité d'un collecteur qui devait, dans l'architecture précédente, contourner la bride de fixation entre chambre ventriculaire et chambre d' actionnement pour y amener le fluide d'actionnement. Dans la nouvelle architecture proposée, où on a inversé les positions des chambres ventriculaires et d'actionnement, les actionneurs disposés au centre refoulent directement le fluide dans les chambres d' actionnement ; les pertes de charge sont diminuées, et la quantité totale de fluide d'actionnement nécessaire est plus faible et facile à confiner au centre du coeur. De plus, par rapport à l'architecture connue, il n'est plus nécessaire de prévoir une crépine de protection du sac réservoir souple qui enferme l'huile de fonctionnement et qui était jusque là prévue pour éviter l'aspiration intempestive du sac par les pompes.

Un autre avantage du coeur nouveau est que toutes les surfaces en contact avec le sang sont faciles à rendre hémocompatibles; en effet, en dehors de la membrane hémocompatible connue de l'art antérieur, toutes les autres surfaces destinées à être en contact avec le sang sont avantageusement réalisées en titane, dont on connaît bien l'excellente hémocompatibilité.

Les chambres ventriculaires du coeur artificiel totalement implantable de l'invention comportent vers l'extérieur des parois bombées sur lesquelles sont disposés les orifices d'éjection et d'admission.

Selon une caractéristique très avantageuse de l'invention, les actionneurs sont disposés sensiblement dans la zone du plan médian de symétrie du V formé par les chambres, leurs axes formant approximativement un angle droit, et les moteurs étant placés vers l'extérieur de l'angle droit et les pompes vers l'intérieur.

Selon l'invention, le coeur artificiel comporte un corps central qui assure une ou plusieurs des fonctions suivantes :
- ventricules droit et gauche contenant le fluide d'actionnement des membranes
- réservoir de fluide d' actionnement
- support et positionnement des groupes motopompe d' actionnement
- collecteur de fluide des pompes
- distributeur de pompe
- support des membranes
- support des capteurs de pression et de position
- support pour la fixation et l'étanchéité du sac souple réservoir de fluide
- support des cartes d'électronique de puissance des moteurs, de l'électronique de conditionnement des capteurs et de l'électronique de commande et de régulation médicale
- support du câble de raccordement électrique avec l'extérieur
- support des chapeaux ventriculaires et de la coque externe

Avantageusement, le corps central, réalisé de préférence en matière plastique, supporte les actionneurs et définit deux cuvettes inclinées en V dont le bord retient ladite membrane mobile surmontée par ladite paroi bombée réalisée en titane, chaque cuvette formant avec la membrane une chambre d'actionnement alimentée en huile par les actionneurs dont la pompe est interposée entre l'espace intérieur de ladite chambre et un espace de confinement d'huile formé dans et autour du corps central et limité par un sac réservoir souple, l'ensemble étant enfermé dans une coque rigide en titane.

La partie centrale équipée des groupes d'actionnement, des capteurs, des membranes et de l'électronique noyés dans le fluide confiné dans un réservoir souple constitue un système d'actionnement autonome intelligent.

La partie biologique constituée d'une coque et de deux chapeaux ventriculaires avec les conduits de raccordement supportant les valves entoure complètement l'actionneur et réalisée en tôle de titane microbillé pour assurer la compatibilité avec le sang et les tissus.

Certaines des caractéristiques avantageuses décrites dans le document FR-A-2 625 903 sont ou peuvent être reprises dans l'architecture nouvelle de l'invention :
- séparation des domaines sanguins et des domaines destinés à l'actionnement et à la régulation
- actionnement électrohydraulique constitué d'un moteur à courant continu sans balai et d'une pompe volumétrique à engrenage intérieur à éjection radiale
- utilisation d'une membrane complexe assurant la compatibilité avec le sang et le fluide d' actionnement
- indépendance des débits des ventricules droit et gauche
- raccordement entre la prothèse et les oreillettes réalisé par un raccord rapide en forme de lunette
- hémocompatibilité et biocompatibilité assurées par des surfaces poreuses de matériaux biocompatibles
- régulation des débits assurée par une variation de vitesse des moteurs électriques commandés par un système électronique numérique reproduisant en temps réel le comportement du muscle cardiaque à partir de mesures des pressions ventriculaires et des volumes éjectés par les pompes volumétriques

D'autres caractéristiques et avantages de l'invention apparaîtront à la lecture de la description d'un mode de réalisation préféré de l'invention. Il sera fait référence aux dessins annexés sur lesquels :
- la figure 1 est une vue éclatée des différents ensembles fonctionnels du coeur artificiel,
- la figure 2 est une perspective de l'ensemble monté,
- la figure 3 est une vue de dessus de cet ensemble,
- la figure 4 est une coupe centrale IV-IV de la figure 3, parallèle aux axes des orifices auriculaires,
- la figure 5 est une coupe V-V de la figure 3, passant par l'axe des groupes motopompes,

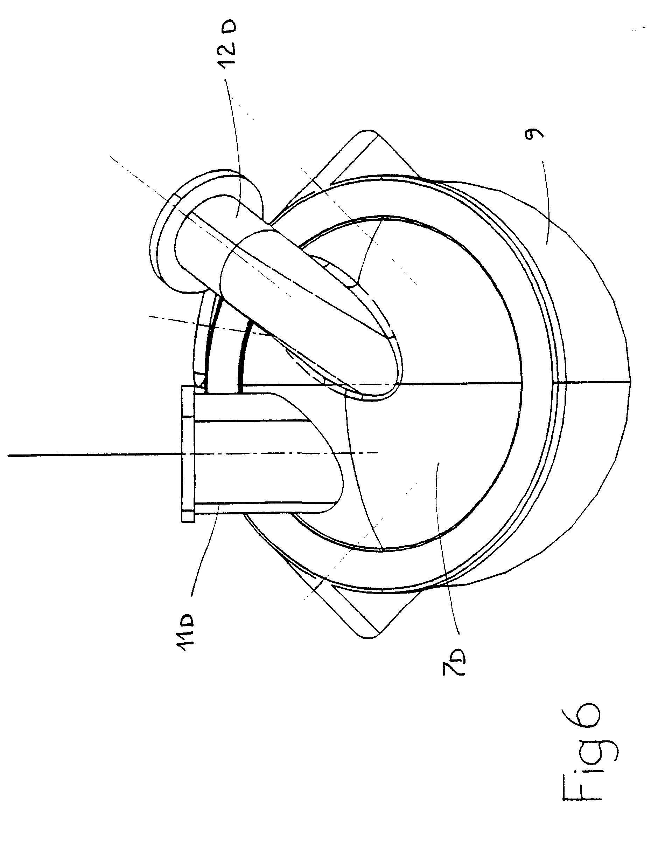

- la figure 6 est une vue de gauche du coeur de la figure 3,
- la figure 7 est une vue de dessus, avec coupe partielle, du corps central,
- la figure 8 représente en perspective axonométrique, le corps central de la figure 7,
- la figure 9 est une coupe de détail au droit d'une pompe d' actionnement,
- la figure 10 est une coupe axiale de détail d'un groupe motopompe d'actionnement.

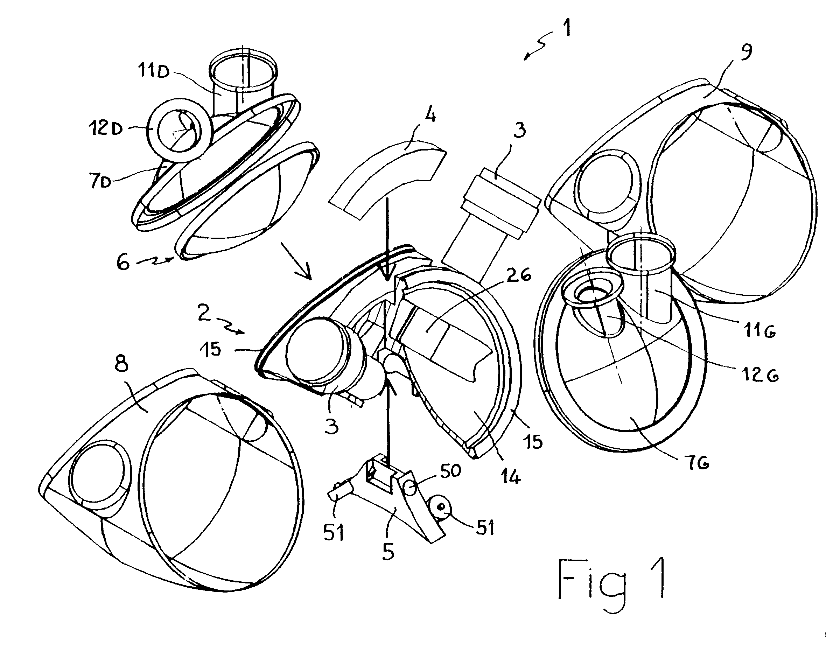

Le coeur artificiel totalement implantable 1 comprend comme le montre la figure 1, un corps central 2 réalisé par exemple en matière plastique et recevant : les deux groupes motopompes inclinés 3 ; la carte électronique 4 de puissance des moteurs sur la partie supérieure ; la carte électronique 5 de commande et des capteurs de pression ventriculaire et de position des membranes vers le bas ; de chaque côté, un ensemble de membrane 6 et un chapeau ventriculaire respectivement gauche 7G et droit 7D muni de conduites sanguines ("gauche" et "droit" se réfèrent aux appellations usuelles pour un coeur humain et sont donc à l'inverse de ce qu'on voit sur la figure). Autour du corps 2 monté, vient s'enfiler le sac souple réservoir 8 et se monter la coque rigide 9.

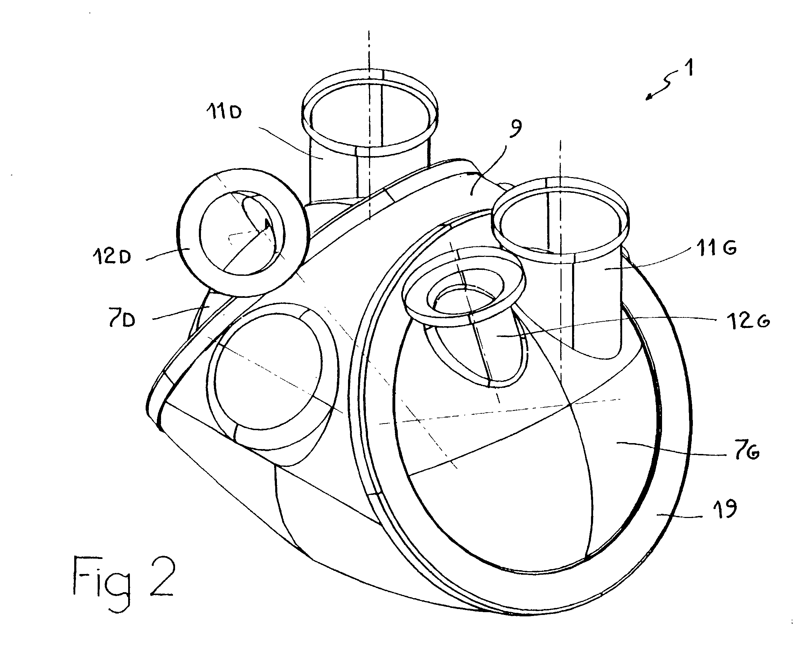

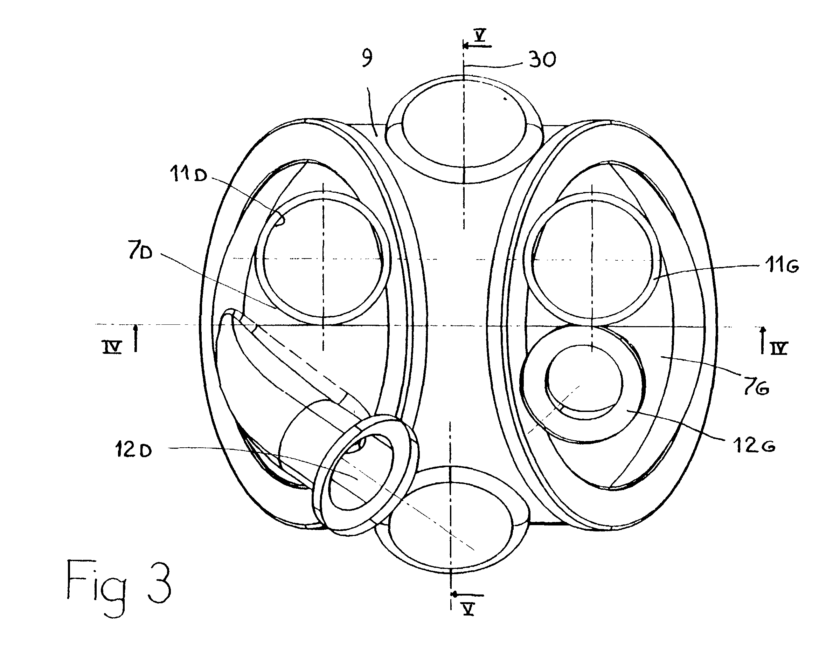

Le coeur 1 entièrement monté (figures 2 et 3) ne montre que des surfaces extérieures en titane, matériau connu pour sa bonne hémocompatibilité. Ce sont les surfaces de la coque 9 et des chapeaux ventriculaires 7G et 7D. La coque 9, en deux parties, est grossièrement en forme de manchon coudé dont les deux sections circulaires d'extrémité sont formées par lesdits chapeaux ventriculaires bombés 7G, 7D, inclinés selon les deux plans d'un dièdre dont l'angle est vers le haut.

La calotte bombée du chapeau gauche 7G comporte une tubulure d'admission 11G destinée à être connectée à l'oreillette gauche et une tubulure d'évacuation 12G destinée à être raccordée à l'aorte.

La calotte du chapeau droit 7D comporte une tubulure d'admission 11D à connecter à l'oreillette droite, et une tubulure d'évacuation 12D à raccorder à l'artère pulmonaire.

Les différentes tubulures 11 et 12, en titane, comportent à l'intérieur de leur orifice les valves nécessaires, comme connu de l'homme de métier. Elles comportent aussi des plateaux et brides de raccordement. Enfin, leur positionnement (écartement et inclinaison) est prévu pour correspondre aussi bien que possible à la localisation des organes naturels auxquelles ils sont amenés à se raccorder.

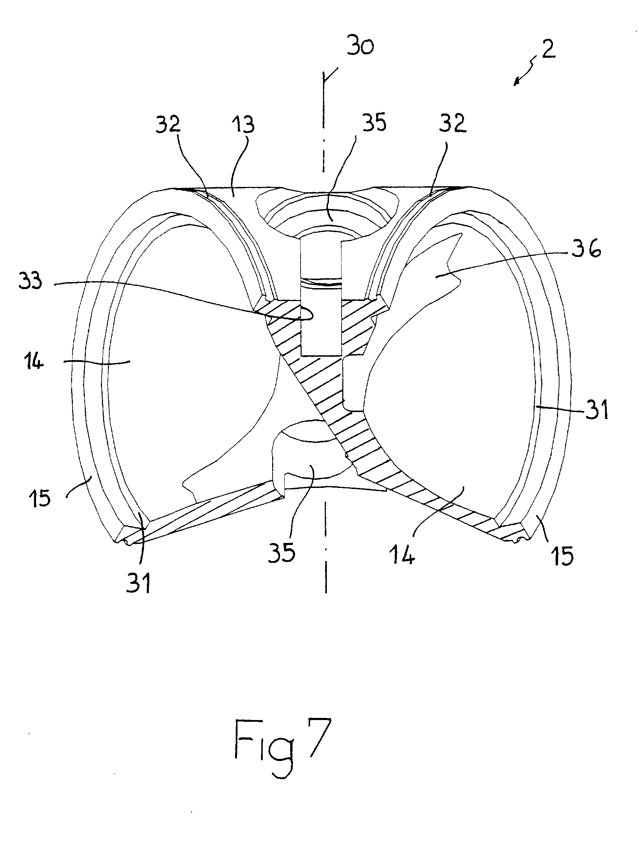

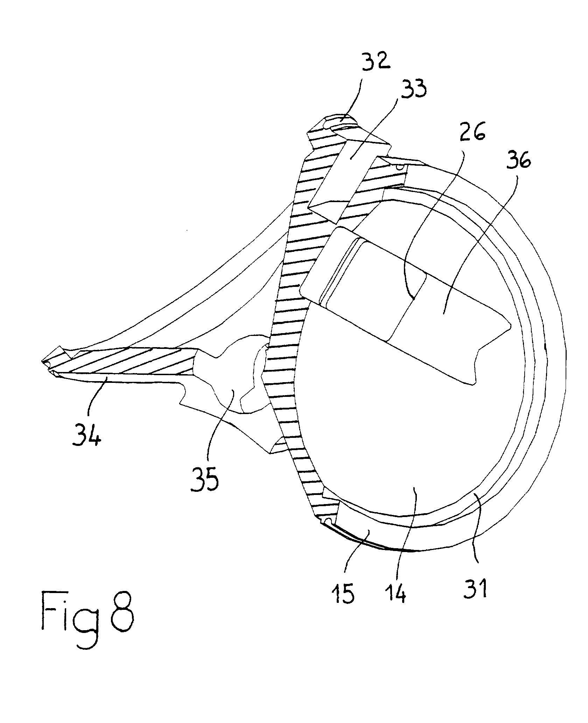

Le corps central 2 (figures 1, 4, 5, 7 et 8) est réalisé en matière plastique, par usinage ou par moulage, sous forme d'un noyau 13 sensiblement symétrique par rapport à un plan médian vertical 30, et définissant latéralement deux coupelles 14 entourées de bords circulaires 15 destinés à recevoir les ensembles de membrane 6 et les bords des chapeaux ventriculaires 7G, 7D.

Un épaulement intérieur 31 sous le bord 15 permet de venir caler l'ensemble de membrane 6, tandis qu'à l'extérieur du bord 15, une gorge périphérique 32 est prévue pour recevoir un joint d'étanchéité lors du montage des brides de fixation 19. Le haut du noyau 13, partie la plus étroite du corps 2 où les coupelles 14 se rapprochent le plus, est dégagé sur le milieu et forme un secteur évidé 33 de réception de la carte électronique 4 de puissance des moteurs. La partie inférieure du noyau, plus large puisque les coupelles 14 sont éloignées, forme également un logement 34 d'insertion de la carte électronique 5 de commande. Le noyau 13 intègre également les trous traversants 35 destinés à loger partiellement les motopompes 3. Les trous 35 sont sensiblement centrés dans le plan médian 30 et forment entre eux un angle avantageusement voisin de 90°. Une fenêtre rectangulaire 26 fait communiquer l'intérieur du trou 35 avec la cuvette 14, à laquelle elle est raccordée par un divergent 36 destiné à ralentir progressivement le fluide.

Chaque ensemble de membrane 6 comprend un cercle de monture 16 dans lequel sont montées les deux membranes souples 17, 18 respectivement mécanique (en matière plastique) et biologique (en péricarde) comme divulgué par le document FR-A-2 625 903. Le cercle 16 vient se loger contre l'épaulement annulaire 31 du bord 15, et est bloqué par le chapeau ventriculaire 7G, 7D et une bride 19. Un joint d'étanchéité 20 est prévu entre la gorge 32 du noyau 13 et la bride 19 fixant le chapeau.

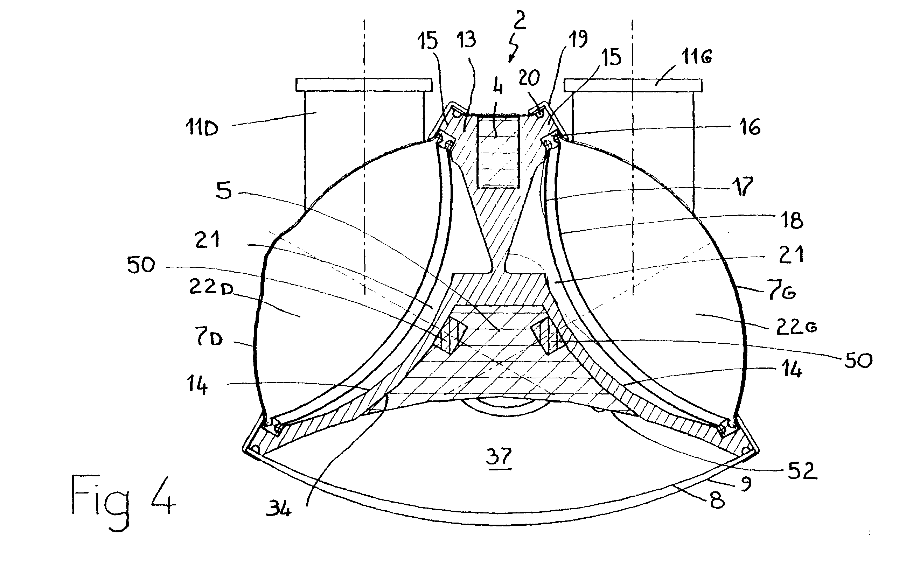

Comme le montre la figure 4, la cuvette 14 du noyau délimite avec la membrane 17 une chambre d'huile 21, tandis que la membrane 18 délimite avec la calotte 7G, 7D une chambre de sang 22G, 22D correspondant respectivement aux ventricules gauche et droit du coeur, en communication fluidique avec les tubulures 11 et 12.

L'actionnement des membranes est assuré par les motopompes 3, logées sur le noyau 13 entre les cuvettes 14 opposées des chambres d'huile.

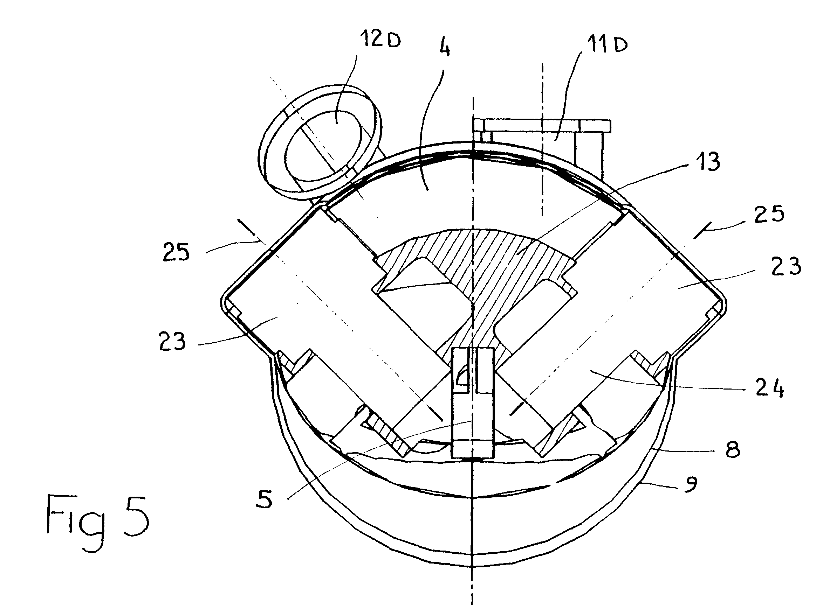

Chaque motopompe 3 comporte une partie de moteur 23 et une partie de pompe 24 disposées sur un même axe 25. Les deux axes 25 des deux motopompes sont sensiblement dans le plan médian 30 de symétrie de noyau 13, et forment entre eux un angle avantageusement voisin de 90°, comme on le voit figure 5.

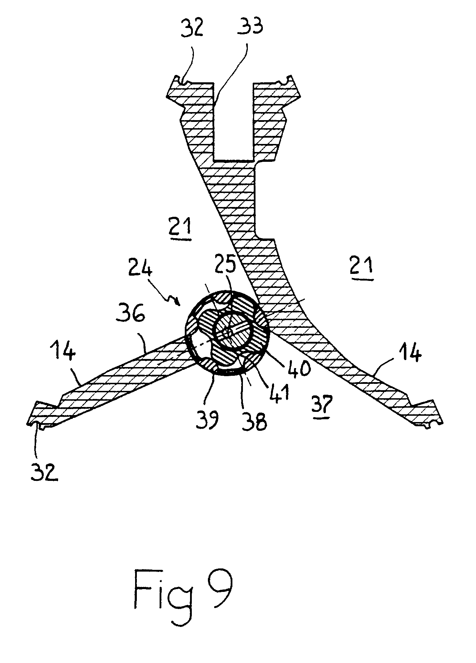

La pompe 24 est une pompe volumétrique à engrenages intérieurs à aspiration et refoulement radial. La pompe 24 communique d'un côté avec la chambre d'actionnement 21 grâce à la fenêtre 26 formée dans la cuvette 14, et de l'autre côté avec l'espace interne 37 rempli de fluide de transmission (avantageusement huile minérale ou huile siliconée), comme on le voit sur la figure 9. Cette figure montre la couronne extérieure 38 à denture intérieure composée de n lobes 39 dans lesquels engrènent n-1 lobes 40 de l'élément intérieur excentrique 41. La couronne 38 tourne sur l'axe 25 et un système d' entraînement connu en soi est prévu pour l'élément intérieur 41. Les lobes 39 de la couronne extérieure 38 sont suffisamment écartés pour permettre un large passage du fluide radialement, sans pertes de charge excessives.

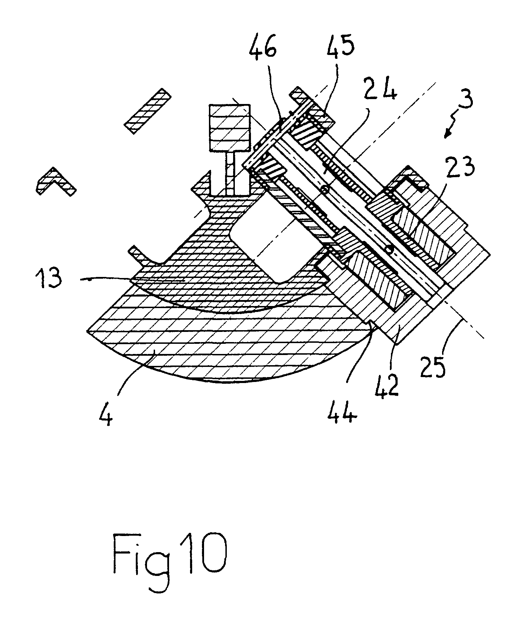

La figure 10 montre l'implantation axiale du groupe motopompe 3 dans le noyau 13 du corps central. Le stator 42 est collé sur le noyau 13 et positionné axialement par un épaulement 44 du stator 42. On vient introduire le rotor du groupe motopompe 24 dans le stator 42 par l'intérieur, et on le ferme avec le couvercle 45 qu'on bloque par une goupille 46.

Les moteurs 23 sont des moteurs à courant continu sans balai dont l'électronique de puissance est rassemblée dans la carte 4.

L'électronique de commande est rassemblée dans la carte inférieure 5. Celle-ci loge aussi des capteurs 50 à effet Hall qui coopèrent avec un aimant placé sur la membrane mécanique 17 pour en détecter la position où elle est proche du fond de la cuvette 14 (position diastolique où la chambre biologique 22 a le volume maximum). Cette détection de fin de course permet de contrôler le volume mesuré de manière connue directement par des capteurs intégrés dans la pompe volumétrique, de manière à recaler la mesure pour éliminer les dérives dues aux fuites inhérentes à la pompe. D'autre part, la carte 5 supporte aussi des capteurs 51 de pression d'huile et de pression du sang.

Comme le montre la figure 4, le profil de la surface inférieure 52 de la carte 5 est prévu sensiblement symétrique de celui de la coque 9, à sa partie inférieure, de manière à offrir au sac souple 8 une fibre neutre de même longueur (dans le plan de la figure) lorsqu'il prend sa position de volume minimum (toute l'huile du volume 37 est refoulée dans l'une ou l'autre des chambres d'huile 21), et minimiser son plissement dans ce sens. On notera que grâce à l'appui offert par ladite surface inférieure 52, le sac souple 8 ne risque pas d'être aspiré par les pompes des groupes 3 et qu'une crépine n'est pas nécessaire.

Le montage du coeur artificiel de l'invention se fait de la manière suivante. Après fabrication du noyau 13 du corps central 2, on colle le stator du moteur 3, et enfile les rotors des motopompes 24 comme expliqué ci-dessus. On place les capteurs de pression et de position puis les cartes électroniques 4 et 5, et on réalise les connexions nécessaires. On enfile le sac réservoir 8 autour du corps central 2, on vient alors placer les ensembles 6 à membrane, puis les chapeaux 7 de chaque côté du corps 2. On fixe la coque en deux parties autour de l'ensemble par deux brides. On n' a pas représenté sur les dessins l'évent d'aération prévu dans la coque 9, ni les connexions électriques reliant les cartes électroniques 4 et 5 à une source d'alimentation électrique extérieure.

## Revendications

1. Coeur artificiel totalement implantable comportant dans un module monocoque deux chambres ventriculaires indépendantes (22G, 22D) à membranes agencées en V et pourvues chacune de deux orifices (11G, 11D, 12G, 12D) orientés vers le haut et munis de valve respectivement pour l'éjection et l'admission du sang, chaque chambre (22G, 22D) ayant son volume variable grâce à une membrane mobile (17, 18) constituant une paroi de la chambre (22G, 22D) et actionnée hydrauliquement par un fluide entraîné par un actionneur (3) comprenant un moteur (23) et une pompe volumétrique (24)
**caractérisé en ce que**
les chambres ventriculaires (22G, 22D) sont agencées de manière à former un V renversé pointant vers le haut en position d'implantation, entre les branches duquel sont disposés lesdits actionneurs (3) et l'espace (37) destiné au fluide d'actionnement.

2. Coeur artificiel selon la revendication 1, **caractérisé en ce que** les chambres (22G, 22D) comportent vers l'extérieur des parois bombées (7G, 7D) sur lesquelles sont disposés les orifices d'éjection et d'admission (11G, 11D, 12G, 12D).

3. Coeur artificiel selon l'une quelconque des revendications 1 ou 2, **caractérisé en ce que** les actionneurs (3) sont disposés sensiblement dans la zone du plan médian (30) de symétrie du V formé par les chambres, leurs axes (25) formant approximativement un angle droit, et les moteurs (23) étant placés vers l'extérieur de l'angle droit et les pompes (24) vers l'intérieur.

4. Coeur artificiel selon l'une quelconque des revendications précédentes, **caractérisé en ce qu'**il comporte un corps central (2), supportant les actionneurs (3) et définissant deux cuvettes inclinées en V dont le bord retient ladite membrane mobile (17, 18) surmontée par ladite paroi bombée réalisée en titane, chaque cuvette (14) formant avec la membrane (17, 18) une chambre d'actionnement (21) alimentée en huile par les actionneurs (3) dont la pompe (24) est interposée entre l'espace intérieur de ladite chambre (21) et un espace (37) de confinement d'huile formé dans et autour du corps central (2) et limité par un sac réservoir souple (8), l'ensemble des éléments précédents étant enfermé dans une coque rigide (9) en titane.

5. Coeur artificiel selon la revendication 4, **caractérisé en ce que** le corps central (2) comporte des logements de réception d'une carte électronique (4) de puissance des moteurs et d'une carte électronique (5) de commande et de capteurs.

6. Coeur artificiel selon l'une quelconque des revendications 4 ou 5, **caractérisé en ce que** le corps central (2) est réalisé en matière plastique.

## Claims

1. A wholly implantable artificial heart comprising within a single-piece module (2) two independent ventricular chambers with membranes (22G, 22D) arranged in a V and each provided with two orifices (11G, 11D, 12G, 12D) oriented towards the top and equipped with valve means respectively for the expulsion and admission of blood, each chamber (22G, 22D) having a variable volume by means of a movable membrane (17, 18) forming a wall of the chamber (22G, 22D) and hydraulically actuated by a fluid driven by an actuator (3) comprising a motor (23) and a volumetric pump (24)
**characterized in that**
said ventricular chambers (22G, 22D) are arranged so as to form an upside down V pointing towards the top, when implanted between the branches of which are arranged said actuators (3) and the space (37) for the actuating fluid.

2. Artificial heart according to claim 1, **characterized in that** the chambers (22G, 22D) comprise on the outside curved walls (7G, 7D) on which are disposed the expulsion and admission orifices (11G, 11D, 12G, 12D).

3. Artificial heart of according to any one of claims 1 or 2, **characterized in that** the actuators (3) are arranged substantially in the zone of the median symmetry plane (30) of the V formed by the chambers, the axes thereof (25) forming approximately a right angle, and the motors (23) being located outside of the right angle and the pumps (24) inside said right angle.

4. Artificial heart according to any one of the preceding claims, **characterized in that** it comprises a central body (2) supporting said actuators (3) and defining two cups inclined in a V configuration the edge of which retains said movable membrane (17, 18) surmounted by said curved wall made of titanium, each cup (14) forming with the membrane (17, 18) an actuating chamber (21) supplied with oil by the actuators (3) whose pump (24) is interposed between the internal space of said chamber (21) and an oil confinement space (37) formed in and around the central body (2) and limited by a supple bag reservoir (8), all of the preceding elements being enclosed in a rigid titanium body shell (9).

5. Artificial heart according to claim 4 **characterized in that** the central body (2) comprises housings for receiving an electronic circuit board (4) for driving motors and a control and sensor electronic circuit board (5).

6. Artificial heart according to any one of claims 4 or 5, **characterized in that** said central body (2) is made of plastics material.

## Patentansprüche

1. Künstliches Herz für Totalimplantation, umfassend in einem einschaligen Modul zwei unabhängige Ventrikulärkammern (22G, 22D) mit V-förmig angeordneten Membranen und mit jeweils zwei Öffnungen (11G, 11D, 12G, 12D), die nach oben gerichtet und jeweils mit Ventil für das Ausstoßen bzw. das Einlassen von Blut versehen sind, wobei das Volumen jeder Kammer (22G, 22D) veränderbar ist, dank einer beweglichen Membran (17, 18), die eine Wand der Kammer (22G, 22D) bildet und hydraulisch durch ein Fluid betätigt ist, welches durch ein Betätigungsgerät (3) zugeführt wird, welches einen Motor (23) und eine volumetrische Pumpe (24) aufweist,
**dadurch gekennzeichnet,**
**dass** die Ventrikulärkammern (22G, 22D) derart angeordnet sind, dass sie ein umgekehrtes V bilden, das in der implantierten Lage mit der Spitze nach oben weist und zwischen dessen Schenkeln die genannten Betätigungsgeräte (3) und der für das Betätigungsfluid bestimmte Raum (37) angeordnet sind.

2. Künstliches Herz nach Anspruch 1, **dadurch gekennzeichnet, dass** die Kammern (22G, 22D) nach außen gewölbte Wände (7G, 7D) aufweisen, an denen die Ausstoß- und Einlassöffnungen (11G, 11D, 12G, 12D) angeordnet sind.

3. Künstliches Herz nach einem der Ansprüche 1 oder 2,
**dadurch gekennzeichnet, dass** die Betätigungsgeräte (3) im wesentlichen im Bereich der mittleren Symmetrieebene (30) des von den Kammern gebildeten V angeordnet sind, wobei ihre Achsen (25) annähernd einen rechten Winkel bilden und die Motoren (23) zur Außenseite des rechten Winkels hin und die Pumpen (24) zu dessen Innenseite hin angeordnet sind.

4. Künstliches Herz nach einem der vorangehenden Ansprüche,
**dadurch gekennzeichnet, dass** es einen Zentralkörper (2) aufweist, der die Betätigungsgeräte (3) trägt und zwei V-förmig geneigte Wannen begrenzt, deren Rand die bewegliche Membran (17, 18) festhält, über welcher sich die aus Titan bestehende gewölbte Wand befindet, wobei jede Wanne (14) mit der Membran (17, 18) eine Betätigungskammer (21) bildet, die durch die Betätigungsgeräte (3) mit Öl versorgt wird, deren Pumpe (24) zwischen dem Innenraum der Kammer (21) und einem Ölvorratsraum (37) angeordnet ist, der in dem Zentralkörper (2) und um diesen herum gebildet ist und von einem weichen Vorratssack (8) begrenzt wird, wobei die Gesamtheit der vorgenannten Elemente in einer starren Schale (9) aus Titan eingeschlossen ist.

5. Künstliches Herz gemäß Anspruch 4, **dadurch gekennzeichnet, dass** der Zentralkörper (2) Schlitze für die Aufnahme einer elektronischen Karte (4) für die Modulleistung und einer elektronischen Steuerkarte (5) sowie von Messfühlern aufweist.

6. Künstliches Herz nach einem der Ansprüche 4 oder 5,
**dadurch gekennzeichnet, dass** der Zentralkörper (2) aus Kunststoff besteht.
